# EUROPÄISCHE PATENTSCHRIFT

(11) **EP 1 703 844 B1**
(45) Veröffentlichungstag und Bekanntmachung des Hinweises auf die Patenterteilung: **21.11.2007**
(21) Anmeldenummer: 05715053.4
(22) Anmeldetag: 03.03.2005
(51) Int. Cl.: A61B 10/00

(54) **Vorrichtung zur Entnahme und zum Ausstrich von Zellen**
DEVICE FOR REMOVING AND SMEARING CELLS
DISPOSITIF DE PRELEVEMENT ET D'ETALEMENT DE CELLULES

(30) Priorität: 03.03.2004 DE 102004010983
(43) Veröffentlichungstag der Anmeldung: 27.09.2006
(73) Patentinhaber: Georg-August-Universität Göttingen, 37075 Göttingen (DE)
(72) Erfinder: HUSCHMAND, Nia, Abdolhamid, 51545 Waldbröl (DE); WEGENER, Reihold, 37079 Göttingen (DE)
(74) Vertreter: Stornebel, Kai
(86) Internationale Anmeldenummer: PCT/DE2005/000370
(87) Internationale Veröffentlichungsnummer: WO 2005/084555

(56) Entgegenhaltungen:
- EP-A- 0 056 493
- AT-B- 392 411
- CH-A5- 653 880
- US-A- 4 754 764
- US-A- 5 623 941
- US-A1- 2001 022 063
- US-B1- 6 346 086

## Beschreibung

Die Erfindung betrifft eine Vorrichtung zur Entnahme und Ausstrich von Zellen für eine zytologische Untersuchung mit einem Griff, an dessen vorderem Ende eine Einrichtung zur Aufnahme der Zellen angeordnet ist. Insbesondere ist eine solche Vorrichtung geeignet, Untersuchungen des Gebärmutterhalses vorzunehmen, insbesondere einen Abstrich aufzunehmen.

Aus dem Stand der Technik sind verschiedene Möglichkeiten bekannt, einen Zellabstrich vorzunehmen, insbesondere bei Vorsorgeuntersuchungen für Zervixkarzinome. Eine Möglichkeit zur Aufnahme der Zellen besteht in der Verwendung eines Watteträgers, bei dem an einem Ende eines Holzstabes Watte angeordnet ist. Durch einen Watteträger wird erfahrungsgemäß eine geringe Läsion gesetzt, darüber hinaus wird eine tiefe, endozervikale Zellentnahme ermöglicht. Nachteilig bei der Verwendung eines Watteträgers ist die Tatsache, dass für einen Abstrich zwei Watteträger benötigt werden und dass der Ausstrichdruck in der Regel ungleichmäßig ist. Ein zu starker Ausstrichdruck führt zu erheblichen Zell- und Kerndegenerationen, ein zu geringer Ausstrichdruck führt zu einer zu geringen Zellentnahme.

Eine weitere Methode oder Vorrichtung zur Abstrichentnahme besteht in einem Szalay-Spatel, der mit einem Griff ausgebildet ist, an dessen vorderem Ende ein Abschnitt zur endozervikalen Zellaufnahme ausgestattet ist.

Diesem Abschnitt, vom Griff aus gesehen, ist ein Absatz vorgelagert, der die exozervikalen Zellen aufnimmt. Der Szalay-Spatel ermöglicht eine größere Zellausbeute gegenüber dem Watteträger sowie eine gleichzeitige Entnahme und Ausstrich endozervikaler und ektozervikaler Zellen.Nachteilig ist jedoch, dass eine tiefe, endozervikale Entnahme nicht immer möglich ist und bei schräggestellter Zervix der hintere Teil der Zervix nicht sicher zu erfassen ist. Darüber hinaus besteht das Risiko der Verletzung der Zervixoberfläche.

Der sogenannte "Cytobrush" besteht aus einem Griff mit am vorderen Ende angeordneter Bürste, mit der auch bei einem engen Zervikalkanal die Entnahme der Zellen bei einer hohen Ausbeute gelingt. Nachteilig daran ist die Gefahr, dass vitale Drüsenzellen aus ihrem Verband gerissen werden, wodurch Fehlinterpretationen möglich werden. Darüber hinaus kann eine Blutung verursacht werden, was zu einer eingeschränkten Beurteilbarkeit der entnommenen Zellen führt.

Schließlich existiert der sogenannte "Zervexbrush", bei der an dem vorderen Ende eines Griffes der Zervixkontur angepasste Bürstenelemente angeordnet sind, mit denen gleichzeitig endo- und ektozervikale Zellen entnommen werden können. Nach der Entnahme wird der Bürstenkopf in das Labor geschickt, wo die weitere Aufarbeitung und der Ausstrich auf einen Objektträger erfolgt. Diese Vorrichtung ist sehr teuer und als Ausstrichinstrument ungeeignet.

Aus der DE 21 35 477A1 ist ein cytologischer Probenentnehmer bekannt, bei dem auf einer an einem Griff befestigten Platte ein Schaumstoffkegel aus Polyurethanschaum aufgeklebt ist. Beim Einführen des Kollektorteiles wird der Kegel zusammengepresst, was eine Probenentnahme im hinteren Zervixkanal verhindert. Ebenfalls tritt eine Verformung des Kegels bei dem Abrollen auf dem Objektträger auf, was ein kontrolliertes und gleichmäßiges Auftragen der Zellen auf den Objektträger verhindert und somit das Risiko eines falschen Befundes bei der Auswertung der Zellen erhöht bzw. eine Auswertung nicht ermöglicht. Ebenso ist eine Zuordnung der Zellen zu einem Entnahmeort innerhalb des Zervixkanales nicht möglich. Die Vorrichtung ist ungeeignet und unbrauchbar für einen zervikalen Ausstrich.

Die US 4,754,764 beschreibt eine bürstenartige Ausgestaltung einer Zellaufnahmeeeinrichtung. Ein konusartiger Endozervikalteil hat einen proximalen Abschluss mit einem exozervikalen Bürstenteil in Gestalt einer Scheibe. Alternativ zu einer Ausgestaltung als Bürste können die Borsten auch schwammartig aus einem geschlossenporigen Schaumstoff ausgebildet sein.

Die US 6,346,086 B1 beschreibt eine endozervikale und exozervikale Zellaufnahmeeinrichtung mit einem aufsteckbaren Kopf, der je nach Anwendungsgebiet, unterschiedliche Konturen aufweist, beispielsweise kalottenförmig, zylindrisch oder konisch mit einer zylindrisch hervorstehenden, abgerundeten Spitze.

Die EP 056 493 A1 beschreibt ein gynäkologisches Abstrichinstrument für die Früherkennung von Krebszellen am Muttermund. Es besteht aus einer an einem Haltestab drehbar gelagerten Aufnahmerolle mit geschlossener Oberflächenstruktur. Die Oberfläche der Rolle ist vorzugsweise um einen Winkel von ca. 105° gegen die Längsachse des Haltestabes geneigt.

Aufgabe der vorliegenden Erfindung ist es, eine Vorrichtung bereitzustellen, die die Nachteile des Standes der Technik überwindet und eine preiswerte Lösung für eine exakte Zellaufnahme und eine zuverlässige Lösung für den Ausstrich auf einen Objektträger bereitstellt.

Erfindungsgemäß wird diese Aufgabe durch eine Vorrichtung mit den Merkmalen des Anspruchs 1 gelöst, nämlich, indem die Einrichtung als ein sich nach vorne verjüngender Konus ausgebildet ist, der sich dem Zervikalkanal und der Portio anpasst und bei der Zellentnahme sowohl intrazervikale Zellen als auch Zellen der Portiooberfläche abstreicht. Bei der Probenentnahme ist die Durchführung einer Drehbewegung erforderlich, da bei der gynäkologischen Anwendung räumlich beengte Verhältnisse herrschen; insbesondere sind die möglichen Freiheitsgrade für die Probenentnahme limitiert. Die Drehbarkeit ist aufgrund des minimalen Raumangebotes im Arbeitsbereich notwendig, um eine optimale Zellentnahme zu garantieren. Durch die Anordnung einer Stabilisierung innerhalb der Einrichtung ist eine präzise Zellentnahme bei gleichzeitiger Gewährleistung einer räumlichen Zuordbarkeit der ausgestrichenen Zellen auf dem Objektträger gegeben, da es weder bei der Zellentnahme noch bei dem Ausstrich zu einer nennenswerten Längendeformation der Entnahmeeinrichtung kommt.

Die Erfindung sieht vor, dass die Einrichtung zur Aufnahme der Zellen relativ zu dem Griff drehbar angeordnet ist. Dadurch wird ermöglicht, dass eine schonende Zellaufnahme an der zu untersuchenden Stelle stattfindet, gleichzeitig werden die Zellen durch das Abrollen der Einrichtung zur Aufnahme der Zellen auf einem Objektträger gleichmäßig auf diesem abgelegt, wodurch sich eine gleichmäßige und unverfälschte Begutachtung der aufgenommenen Zellen ermöglichen lässt. Es entfällt das Erfordernis, dass der Griff mitgedreht werden muss, was zu Verschmierungen bei ungenauer Durchführung führt.

Zudem ist vorgesehen, dass an der Außenseite der Einrichtung zur Aufnahme der Zellen eine Schaumstoffschicht angeordnet ist, auf der sich eine hohe Ausbeute an Zellen anlagern kann. Als optimaler Schaumstoff wurde bei Versuchen ein Material aus Polyurethanschaum mit einer Rohdichte von 24kg/m³, einer Zugfestigkeit von mehr als 110 KPa, einer Bruchdehnung von mehr als 120%, einer Stauchhärte bei 40% Verformung von 4,0 KPa, einer Druckverformung von weniger als 10% und einer Porenzahl von 1,18 bis 1,5 ppmm (Poren pro Millimeter) ermittelt. Durch den Schaumstoff wird gleichzeitig die Gefahr einer Verletzung der untersuchten Stelle, üblicherweise ein schleimhautbehaftetes Gewebe, vermieden.

Mit Vorteil besteht die Einrichtung bzw. der Konus vollständig aus Kunststoff, was eine größere Verformbarkeit des Konus bewirkt, wodurch sich die Einrichtung sehr gut an den Zervikalkanal und der Portio anpassen kann. Gleichzeitig werden intrazervikale Zellen und Zellen der Portiooberfläche abgestrichen. Aus experimentellen Untersuchungen hat sich gezeigt, dass die optimalen Abmessungen des Schaumstoffkegels bei einer Länge von ungefähr 21 mm bis 23 mm, einem Konusgrunddurchmesser von ca. 14mm bis 16mm und einem Konuswinkel von ungefähr 25° liegen. Der Konus kann leicht bogenförmig ausgebildet sein. Damit konnten die besten Werte bei der Zellentnahme und beim Übertragen auf einen Objektträger erzielt werden.

Erfindungsgemäß ist die Einrichtung zur Aufnahme der Zellen als eine Kappe ausgebildet, die auf einem Träger angeordnet oder befestigt ist, so dass die Kappe aus einem zellaufnehmenden oder zelltragenden Stoff einfach hergestellt werden kann. Vorteilhafterweise wird die Kappe aus Polyurethanschaum gemäß der oben genannten Werte oder aus ähnlichen Materialien mit ähnlichen Werten oder Eigenschaften hergestellt. Der Träger als solcher wird getrennt hergestellt, wobei die Kappe auf den Träger aufgesteckt oder aufgeklebt werden kann. Bevorzugt wird die Kappe auf eine Spitze am vorderen Ende aufgesteckt, die auch zur Stabilisierung des Schaumstoffes dient.

Um ein Abrollen des Konus bzw. der Einrichtung auf einem Objektträger nicht zu behindern und eine mögliche Läsion der Probenentnahmestelle durch eine scharfe oder feste Kante an dem proximalen Teil des Trägers zu vermeiden, weist der Träger erfindungsgemäß eine Grundfläche auf, deren Durchmesser kleiner als der Durchmesser der Grundfläche der Einrichtung zur Aufnahme der Zellen bzw. kleiner als der Durchmesser der Konusgrundfläche ist. Der zellaufnehmende oder zelltragende Stoff, insbesondere Schaumstoff, umschließt dabei zumindest den Rand der Konusgrundfläche, einerseits um die Probenentnahmestelle vor Verletzungen zu schützen, andererseits um eine möglichst vollständige und gleichmäßige Zellentnahme zu gewährleisten. Der den Rand der Grundfläche umgebende Stoff oder Schaumstoff wird bei der Zellentnahme komprimiert und begrenzt und vergleichmäßigt dadurch die auf das Gewebe ausgeübte Belastung.

Durch die drehbare Ausbildung ist es zudem möglich, auf einfache Art und Weise einen gleichmäßigen Ausstrich in zwei getrennte Streifen auf dem Objektträger zu erhalten, mit getrennten Bereichen für intrazervikale Zellen und Zellen der Portiooberfläche.

Insbesondere zu Zwecken der Zellaufnahme ist es vorgesehen, dass eine Verriegelungseinrichtung zur drehfesten Lagerung des Konus bzw. der Einrichtung an dem Griff vorgesehen ist. Die Entnahmeeinrichtung kann dadurch leicht um 360° gedreht werden, um Zellen aufzunehmen, wenn die Verriegelungseinrichtung aktiviert ist. Vorteilhafterweise ist die Verriegelungseinrichtung als ein entlang der Grifflängserstreckung verschiebbares Formschlusselement ausgebildet, das in der Verriegelungsstellung in zumindest eine korrespondierend ausgebildete Aussparung eingreift.

Das Formschlusselement ist beispielsweise als Abflachung, Absatz, Vorsprung oder eine Verzahnung, vorzugsweise als eine Verzahnung im Sägezahnprofil ausgebildet, wobei die Formschlusselemente in entsprechende Vorsprünge, Ausnehmungen oder korrespondierend ausgebildete Verzahnungen eingreifen. Für den Fall einer Ausgestaltung der Formschlusselemente als ein Sägezahnprofil kann durch die Stellung der Zähne eine Verriegelungsrichtung in eine Drehrichtung ermöglicht werden, in eine andere Drehrichtung kann die freie Drehbarkeit gewährleistet sein, um die Zellen auf einem Objektträger abzurollen.

Insbesondere bei einer Zellentnahme im Zusammenhang mit der Zervikalkarzinom-Früherkennung wird eine Kraft in Grifflängserstreckung bei der Zellentnahme aufgebracht. Wenn ein Federelement das Formschlusselement in Entriegelungsrichtung belastet, ist eine freie Drehbarkeit der Einrichtung oder des Konus relativ zu dem Griff grundsätzlich gewährleistet, erst bei Aufbringung einer in Axialrichtung wirkenden Kraft greifen die Formschlusselemente in die korrespondierenden Aussparungen ein und verriegeln die Einrichtung drehfest, so dass bei einer Drehung die Einrichtung zusammen mit dem Griff bewegt wird. Über das Federelement kann auch diejenige Kraft eingestellt werden, mit der die Einrichtung gegen den Zervikalkanal oder die Portio gedrückt wird.

Sofern die Einrichtung aus einem Träger mit darauf angeordneter Schaumstoffkappe besteht, ist der Träger relativ zu dem Griff drehbar gelagert und weist vorteilhafterweise entweder ein Formschlusselement oder eine Aussparung auf, um eine Verriegelung des Trägers relativ zu dem Griff zu bewirken.

Aus Gründen der besseren Handhabbarkeit kann der Griff zumindest teilweise einen eckigen Querschnitt oder einen runden Querschnitt mit einer strukturierten Oberfläche aufweisen, damit der Griff und die Einrichtung zur Aufnahme der Zellen besser und leichter gedreht werden können. In Verbindung mit der konischen und damit rotationssymmetrischen Einrichtung zur Zellentnahme stellt ein kantiger Griff, insbesondere in der Ausgestaltung als ein kantiger Stab eine besonders preiswerte und gut handhabbare Lösung dar, mit der sehr gut die Zellentnahme und der Abstrich durchgeführt werden kann. Der kantige Stab ermöglicht oder erleichtert das Aufbringen des notwendigen Drehmomentes bei der drehenden Zellentnahme im Zervikalkanal. Das für die Drehung notwendige Drehmoment ist aufgrund der vollständigen Umschließung der Entnahmeeinrichtung durch den Zervikalkanal recht hoch. Da eine drehende Bewegung erfolgen muß, um eine gute Probenentnahme zu gewährleisten, ist die kantige Ausbildung des Griffes oder Stabes wichtig.

Aus Gründen der Stabilität und zur Optimierung der zu erzielenden Ergebnisse bei der Probenentnahme und beim Abstreichen auf einem Objektträger hat sich erfindungsgemäß eine in den Schaumstoff hineinragende Spitze, die über den Träger beziehungsweise eine Grund- oder Andruckplatte als vorteilhaft erwiesen. Die Spitze stabilisiert der Schaumstoff in Längsrichtung bei der Einführung in die Zervix und während des Entnahmevorganges. Damit wird eine gleichmäßige und optimale Zellentnahme sowohl am Rand der Zervix als auch im Zervixkanal ermöglicht. Ebenso stabilisiert die Spitze den Schaumstoff in Querrichtung beim Abrollen auf einem Objektträger, wodurch ein gleichmäßiges und kontrolliertes Auftragen der Zellen ermöglicht wird und dadurch eine optimale Verteilung der Zellen auf dem Objektträger gewährleistet wird, was zu einer wesentlichen Verbesserung in der Auswertung der Zellen führt.

Nachfolgend werden Ausführungsbeispiele der Erfindung anhand der beigefügten Figuren näher erläutert. Es zeigen:
- Figur 1-: eine erste Variante der Erfindung in perspektivischer Ansicht;
- Figur 2-: eine zweite Variante der Erfindung in Verriegelungs- und Entriegelungsstellung;
- Figur 3 -: eine dritte Variante der Erfindung mit ihren Komponenten und fertig montiert;
- Figuren 4a - 4b -: vergrößerte Darstellungen der Variante nach Figur 3 in Verriegelungs- und Entriegelungsstellung;
- Figur 5 -: ein Anwendungsbeispiel der Vorrichtung gemäß Figur 4;
- Figuren 6a und 6b -: Einzelteildarstellung und geschnittene Darstellung einer vierten Variante der Erfindung;
- Figuren 7a und 7b -: eine montierte Vorrichtung gemäß den Figuren 6a und 6b in Teilschnittdarstellung;
- Figuren 8a - 8e -: eine weitere Variante der Vorrichtung;
- Figuren 9a - 9c -: ein Ausführungsbeispiel mit Detailansichten.

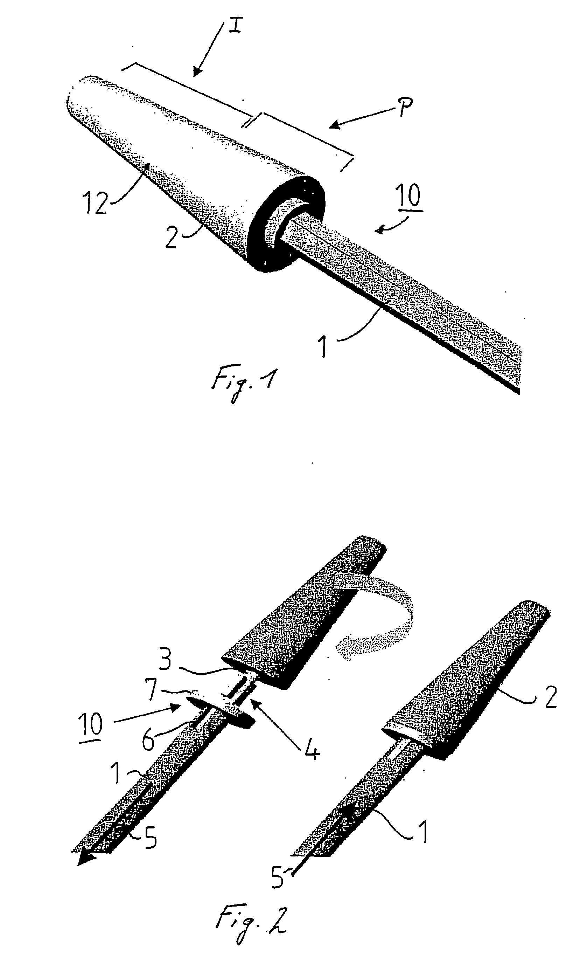

Figur 1 zeigt eine Vorrichtung 10 zur Entnahme und Ausstrich von Zellen für eine zytologische Untersuchung mit einem kantigen, vorliegend im Querschnitt sechseckigen Griff 1 und einer sich konisch nach vorn verjüngenden Einrichtung 2 zur Aufnahme von Zellen. Die Einrichtung 2 weist an ihrer Außenseite 12 eine Schaumstoffschicht auf, an der sich Zellen bei einer Zellentnahme, insbesondere bei einer Zervikalkarzinom-Früherkennung anlagern. In dem vorderen Bereich I werden die endozervikalen Zellen aufgenommen, in dem Griff 1 zugewandten Abschnitt P der Einrichtung 2 werden die Zellen der Portiooberfläche aufgenommen. Die Zellentnahme erfolgt aufgrund der elastischen Schaumstoffschicht gewebeschonend und im Vergleich zu Kunststoffbürsten mit einer höheren Zellausbeute.

In der Figur 2 ist eine Variante und Weiterbildung der erfindungsgemäßen Vorrichtung 10 dargestellt, mit einem Griff 1, in dem ein Träger 3 in Gestalt eines Kunststoffstabes drehbar und in Richtung der Längserstreckung 5 des Griffes 1 verschiebbar gelagert ist. An dem vorderen Ende des Trägers 3 ist eine konische Schaumstoffkappe 2 aufgesteckt. In dem Griff 1 ist eine Ausnehmung 6 ausgebildet, in die eine korrespondierende Verriegelungseinrichtung 4 in Gestalt eines Absatzes eingreifen kann. An dem vorderen Griffende ist eine Andruckplatte 7 angeordnet, um die Schaumstoffkappe abzustützen. In der linken Darstellung der Figur 2 ist die Vorrichtung 10 in Entriegelungsstellung gezeigt, das heißt, dass die Schaumstoffkappe 2 zusammen mit dem Träger 3 um die Längserstreckung 5 des Griffes 1 drehbar ist, wie durch den Pfeil angedeutet, In dieser Entriegelungsstellung kann der Konus 2 leicht auf einem Objektträger abgerollt werden, wodurch sich ein einfacher und gleichmäßiger Ausstrich erhalten lässt. In der rechten Darstellung der Figur 2 ist die Vorrichtung 10 in Verriegelungsstellung dargestellt, das heißt, dass der Griff 1 als äußere Hülle in Richtung auf die Schaumstoffkappe 2 geschoben ist. Das Formschlusselement 4 greift formschlüssig in die Aussparung 6 des Griffes 1 ein und ermöglicht so durch Drehen des Griffes 1 eine Drehung der Schaumstoffkappe 2. Dadurch können Zellen im Gebärmutterhals oder an einem anderen Ort, an dem eine Zellentnahme stattfinden soll, aufgenommen werden.

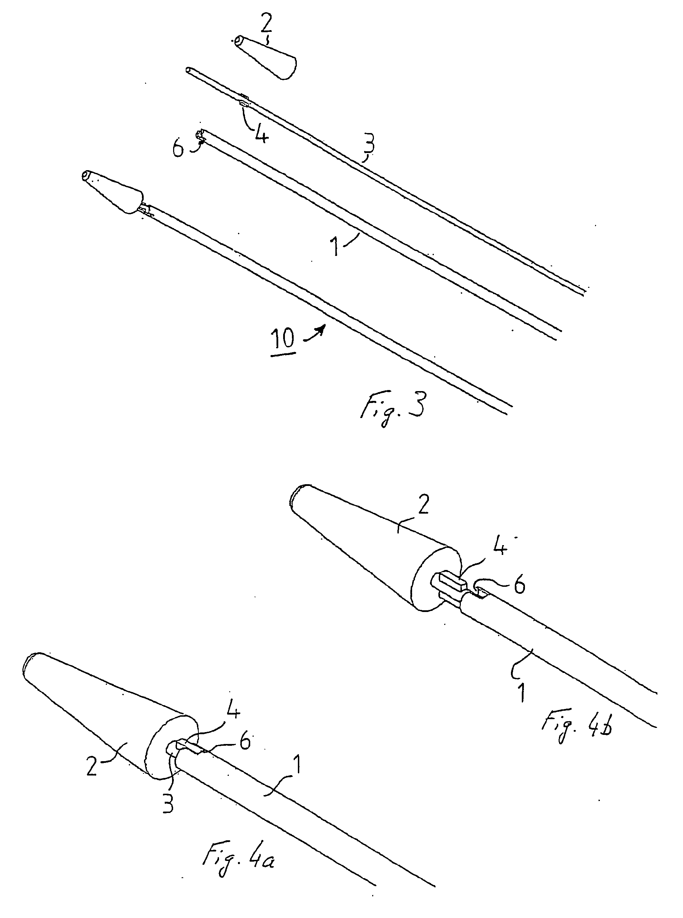

In der Figur 3 ist eine weitere Variante der Erfindung gezeigt, bei der an dem Griff 1 keine Abdruckplatte 7 angeordnet ist. In der Einzelteilzeichnung ist zu erkennen, dass der Träger 3 aus einem langen Stab, vorzugsweise Kunststoffstab besteht, der im vorderen Bereich zwei Formschlusselemente 4 trägt. Diese Formschlusselemente 4 dienen einerseits der drehfesten Verriegelung des Trägers 3 innerhalb des Griffes 1, andererseits als ein Anschlag für die Schaumstoffkappe 2. In dem Griff 1 sind Ausnehmungen 6 in Gestalt von Schlitzen ausgearbeitet, in die die Formschlusselemente 4 des Trägers 3 eingreifen können.

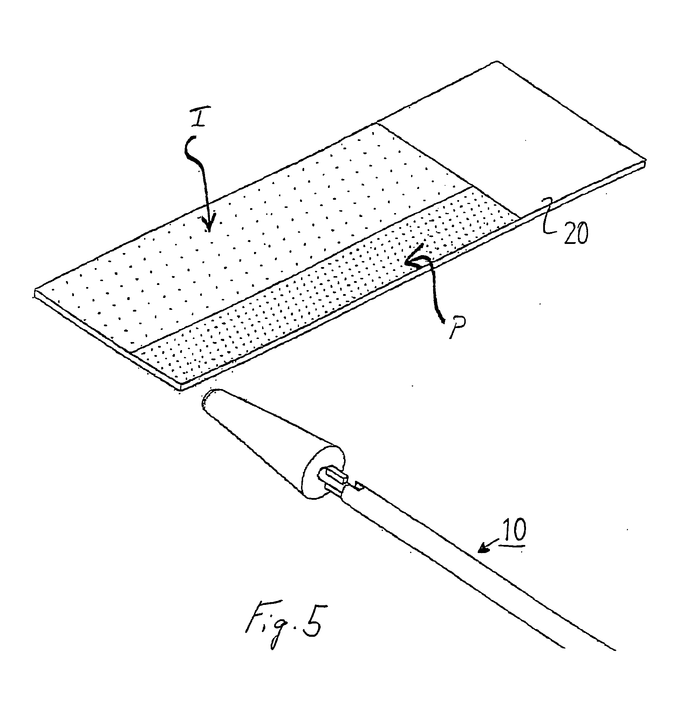

In den Figuren 4a und 4b ist die Variante gemäß der Figur 3 vergrößert dargestellt. In der Figur 4a ist die Vorrichtung in Verriegelungsstellung gezeigt, das heißt, dass die Formschlusselemente 4 des Trägers 3 in die Ausnehmungen 6 des Griffes 1 eingreifen. In dieser Stellung ist eine drehende Zellentnahme möglich. In der Figur 4b ist die Vorrichtung 10 in Entriegelungsstellung gezeigt, in der sehr präzise und einfach ein Ausstrich auf einem Objektträger erfolgen kann.

In der Figur 5 ist angedeutet, wie ein Ausstrich der aufgenommenen Zellen auf einem Objektträger 20 erfolgen kann, nämlich indem die Vorrichtung 10 auf der Oberfläche des Objektträgers 20 entlang geführt wird. Dadurch ist es möglich, dass die endozervikalen Zellen des vorderen Bereiches I des Schaumstoffkonus 2 getrennt von den Zellen der Portiooberfläche P auf dem Objektträger 20 abgelegt werden. Neben der gleichmäßigen und schonenden sowie schnellen Aufbringung der Zellen auf den Objektträger 20 ist durch die präzise Zuordnung der verschiedenen Herkunftsbereiche eine präzise Diagnostik möglich.

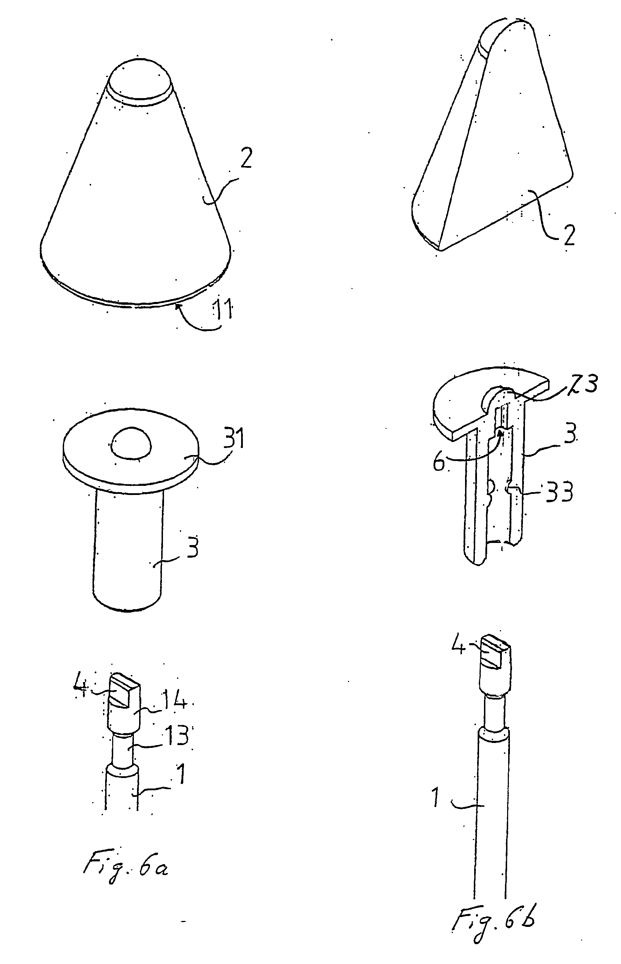

In den Figuren 6a und 6b ist eine Variante der Erfindung dargestellt, bei der der Griff 1 im wesentlichen rund ausgebildet ist und an dem vorderen Ende einen Absatz 13 aufweist, an den sich eine Verdickung 14 anschließt, an der Formschlusselemente in Gestalt von Absätzen 4 angeordnet sind. Auf den Griff 1 wird ein Träger 3 aufgesteckt, der eine vordere Abschlussplatte 31 aufweist. Auf diese Abschlussplatte 31 wird der Schaumstoffkonus 2 aufgesetzt oder aufgeklebt, wobei vorliegend der Schaumstoffkonus 2 vollständig aus Schaumstoff besteht. Alternativ könnte ein Kern aus einem anderen Material von einer Schaumstoffschicht überzogen sein.

In der rechten Figur 6b ist der Träger 3 und die Schaumstoffkappe 2 in Schnittdarstellung gezeigt. Die Schaumstoffkappe ist konisch ausgebildet und besteht aus Schaumstoff, der Träger 3 weist eine Ausnehmung 6 zur Aufnahme des Formschlusselementes 4 am vorderen Ende des Griffes 1 auf. Die Ausnehmung 6 ist als ein korrespondierend ausgebildeter Schlitz 6 ausgebildet. Innerhalb des Trägers 3 sind Vorsprünge 33 oder eine umlaufende Rippe ausgebildet, die in den Absatz 13 des Griffes 1 eingreifen und so ein Abziehen des Trägers 3 von dem Griff 1 erschweren oder unmöglich machen.

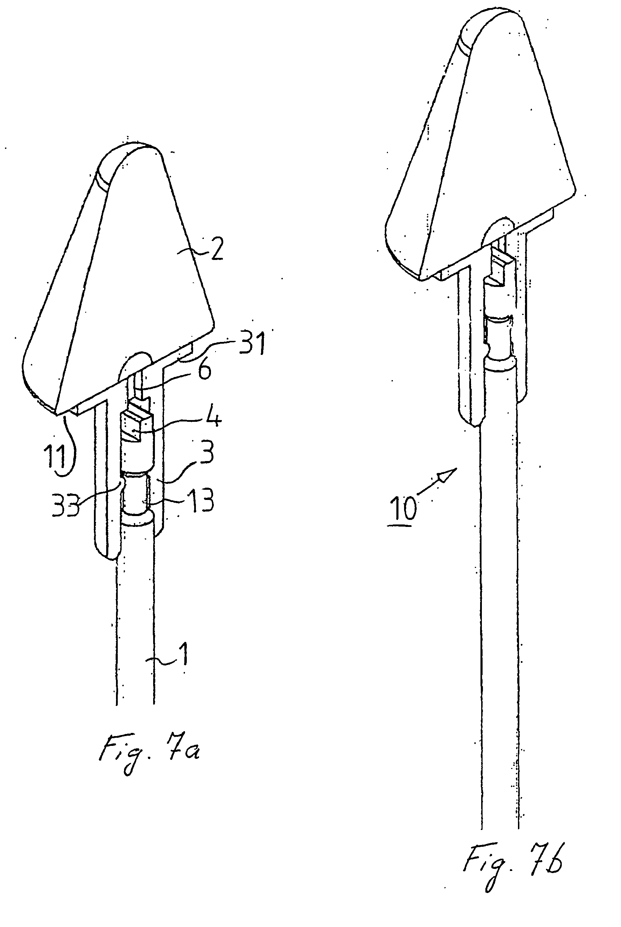

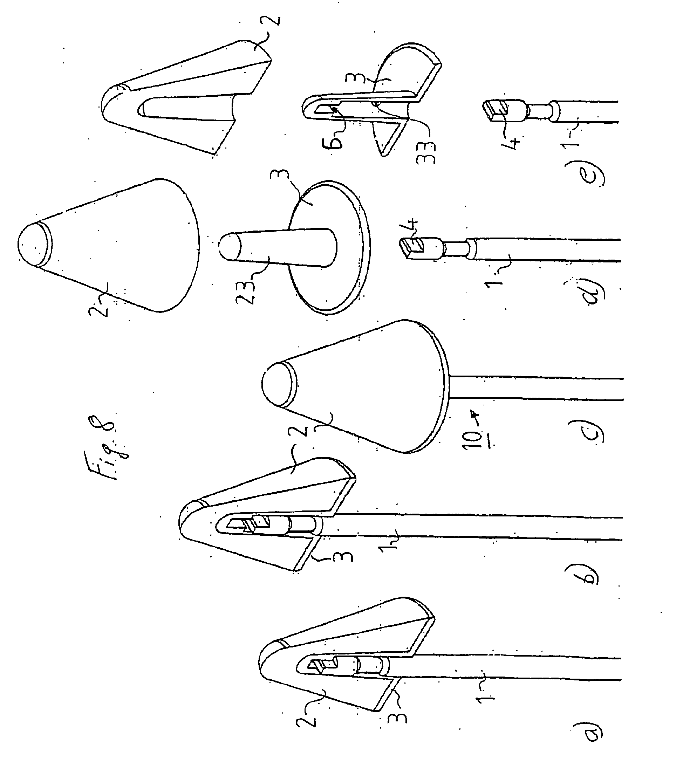

Die Funktionsweise der Vorrichtung ist in den Figuren 7a, 7b gezeigt, in denen die Bauteile der Figuren 6a und 6b montiert dargestellt sind. In der Figur 7a ist der Schaumstoffkonus 2 auf der Abschlussplatte 31 montiert, vorzugsweise festgeklebt, wobei der Durchmesser der Abschlussplatte 31 kleiner als der Durchmesser der Grundfläche 11 des Konus 2 ist. In der Figur 7b ist die Vorrichtung 10 in einer Entriegelungsstellung dargestellt, in der sich sowohl der Konus 2 als auch der Träger 3 frei um den Griff 1 drehen können, da die Formschlusselemente 4 nicht in die Ausnehmung 6 des Trägers 3 eingreifen. Um diesen Zustand aufrecht zu erhalten, kann innerhalb der Ausnehmung 6 eine Feder angeordnet sein, die den Träger 3 von dem Griff 1 wegdrückt. Bei Einführen des Konus 2 in einen Körperhohlraum wird die nicht dargestellte Feder zusammengedrückt und eine Verriegelung bewirkt, so dass eine Drehung des Konus 2 durch die Drehung des Griffes 1 stattfinden kann. In der Figur 7b ist die Vorrichtung 10 in Verriegelungsstellung dargestellt. In der Figur 7 ist zu sehen, dass durch den Vorsprung 33, der in den Absatz 13 eingreift, eine Festlegung des Trägers 3 an dem Griff 1 erfolgt.

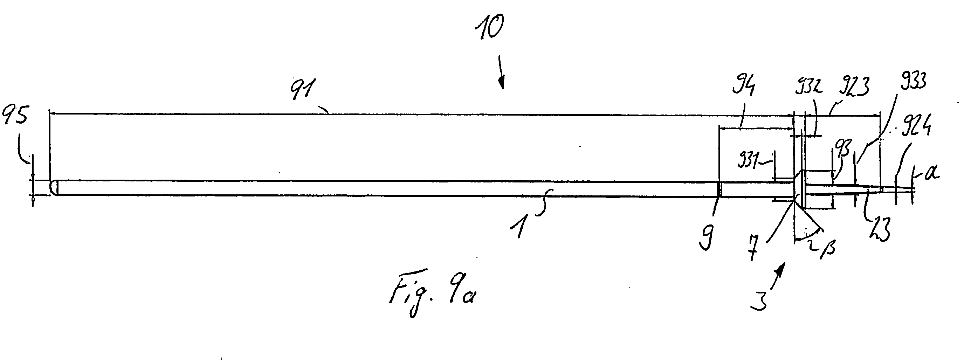

Eine alternative Ausgestaltung des Trägers 3 ist in der Figur 8 dargestellt, wobei in der Figur 8a eine Teilschnittdarstellung in Verriegelungsstellung und in der Figur 8b eine Teilschnittdarstellung in Entriegelungsstellung dargestellt ist. Der Träger 3 ist dabei mit einem kappenförmigen Fortsatz 23 ausgestattet, in dem die Ausnehmung 6 sowie der Vorsprung 33 angeordnet sind. Der Schaumstoffkonus 2 ist nicht massiv oder vollständig aus Schaumstoff ausgebildet, sondern stellt einen Schaumstoffüberzug dar. Die Funktionsweise entspricht ansonsten denen der in der Figur 7 beschriebenen Variante.

Um die Griffigkeit und die Drehbarkeit der Vorrichtung 10 zu erhöhen, ist der Griff 1 eckig, vorzugsweise sechs- oder achteckig ausgebildet. Durch die Ausbildung eines Trägers 3 mit einer Trägerplatte 31 muss eine Arretierung nicht mehr innerhalb des Schaumstoffes selbst lokalisiert sein, was die Herstellung erleichtert, da die mechanischen Elemente durch Urformen, insbesondere Spritzgussformen, hergestellt werden können. Beim Ausstreichen der Zellen wird, wie vorstehend beschrieben, die Verriegelung gelöst und ein Abrollen der Schaumstoffkappe 2 auf einem Objektträger 20 ermöglicht. Durch die Verformbarkeit der Schaumstoffkappe 2 kann eine Zellentnahme an der Portiooberfläche und dem Zervikalkanal in einem Arbeitsgang erreicht werden. Die Oberfläche der Schaumstoff- oder Kunststoffkappe 2 weist feine Poren auf, die eine schonende Zellentnahme bei gleichzeitiger hoher Zellausbeute ermöglicht.

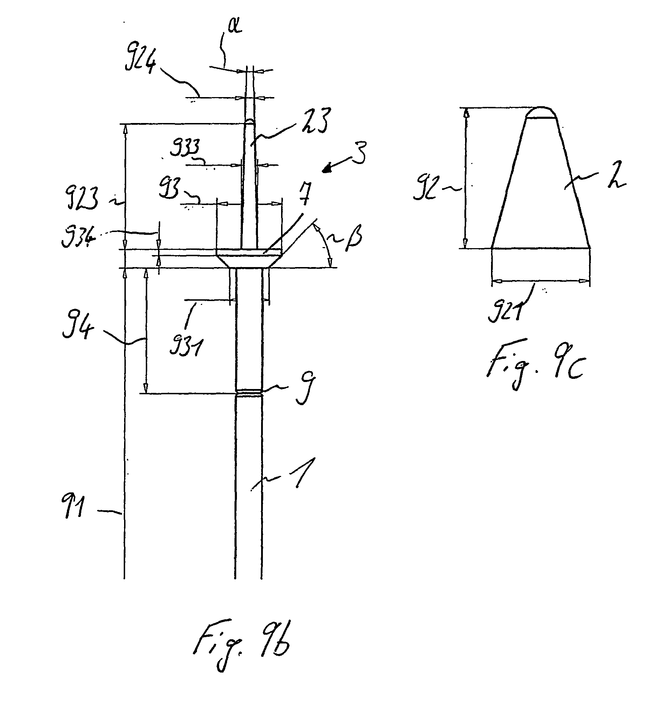

Die Figur 9a zeigt eine einstückig ausgebildete Vorrichtung 10 zur Entnahme und Ausstrich von Zellen mit einem G riff 1, an dem ein Träger 3 mit einer Abdruck- oder Grundplatte 7 angeordn et ist. Nicht dargestellt ist die konische, rotationssymmetrische Schaumstoffkappe, die auf einen kappenartigen Fortsatz 23 aufgesteckt wird. Der Fortsatz 23 dient zur Stabilisierung der Schaumstoffkappe und verhindert ein Zusammendrücken beim Einführen der Vorrichtung 10 in den Zervikalkanal. Dadurch wird es ermöglicht, eine Zellentnahme über die gesamte Außenfläche der Schaumstoffkappe zu erreichen und die gesamte Länge des Zervikalkanals zu erfassen.

Innerhalb des Griffes 1 ist eine Sollbruchstelle 9 ausgebildet, an der der obere Teil der Vorrichtung 10, der als Träger 3 dient, abgeknickt und verpackt zu einem Labor gesendet werden kann. Die Gesamtlänge 91 des Griffes 1 beträgt ungefähr den zehnfachen Wert der Länge des Absatzes 94, an dem der vordere Teil der Vorrichtung 10 mit dem Fortsatz 23 und der Grundplatte 7 angeordnet sind. Eine handhabbare Größe für die Gesamtlänge 91 des Griffes 1 beträgt ungefähr 200 mm, der Griffdurchmesser 95 liegt zwischen 3 mm und 7 mm, vorzugsweise 4 mm.

Die Grundfläche 7 an dem Träger 3 ist in der Ausführungsform gemäß der Figuren 9a und 9b konisch ausgebildet und erweitert sich nach einem Absatz 391, der im Durchmesser um 50 % größer als der Griffdurchmesser ist, in einem Winkel β von 45° bis zum gewünschten Grundplattendurchmesser 93, der in einem Ausführungsbeispiel ungefähr einen Durchmesser von 10 mm aufweist. Ein Rand 934 vergrößert die Anlagefläche der Schaumstoffkappe 2, die in der Figur 9c dargestellt ist und vermindert aufgrund der erhöhten Fläche die Verletzungsgefahr bei der Probenentnahme.

Der sich von der Grundfläche 7 in Längserstreckung des Griffes 1 weiter erstreckende Fortsatz 23 weist eine Länge 923 auf, die der Länge des Absatzes 931 entspricht. Der Durchmesser 933 am Ursprung des Fortsatzes 23 beträgt beispielsweise 2,5 mm, während der Spitzendurchmesser 924 kleiner ist und ungefähr 1,4 mm bis 1,5 mm beträgt. Der Fortsatz 23 verjüngt sich nach vorne in einem Winkel α von ungefähr 3°.

Die in der Figur 9c dargestellte Schaumstoffkappe 2 ist in ihrer Längserstreckung 92 größer als die Länge 923 des Fortsatzes 23, vorzugsweise um ungefähr 10% länger. Der Grunddurchmesser 921 der Schaumstoffkappe 2 ist größer als der Durchmesser 93 der Grundfläche 7, vorzugsweise um 50% größer. Die Schaumstoffkappe 2 kann sich auch über den Rand 934 hinaus in Richtung Griff 1 erstrecken, um das Verletzungsrisiko durch die Grundplatte 7 zu verringern. Die Schaumstoffkappe 2 kann auf den Fortsatz 23 aufgesteckt oder aufgeklebt werden. Durch den Fortsatz 23, der in die Schaumstoffkappe 2 hinein ragt, kann ein funktionswesentliches Einführen der Schaumstoffkappe 2 in den Zervixkanal sichergestellt werden, ohne dass eine axiale Verformung der Schaumstoffkappe 2 auftritt. Die Grundplatte 7 unterstützt die Schaumstoffkappe 2 und trägt zu einer Stabilisierung der Schaumstoffkappe 2 bei Entnahme bei.

Der Griff 1 kann eckig ausgebildet sein, während die Schaumstoffkappe 2 rotationssymmetrisch, vorliegend konisch ausgeführt ist.

## Patentansprüche

1. Vorrichtung zur Entnahme und Ausstrich von Zellen für eine zytologische Untersuchung mit einem Griff (1), an dessen vorderem Ende eine als sich nach vorne verjüngender Konus ausgebildete Einrichtung (2) zur Aufnahme der Zellen angeordnet ist, in der eine in Längserstreckung der Einrichtung (2) wirksame Stabilisierungseinrichtung (23) angeordnet ist, wobei die Einrichtung (2) als eine Kappe ausgebildet ist, die auf einem Träger (3) angeordnet und befestigt ist und der Träger (3) eine Grundfläche aufweist, deren Durchmesser kleiner als der Durchmesser der Grundfläche der Einrichtung (2) ist, wobei die Stabilisierung (23) als eine in die Einrichtung (2) hineinragende Spitze ausgebildet ist, die allseitig von einem Schaumstoff umgeben ist, **dadurch gekennzeichnet, dass** die Einrichtung (2) relativ zu dem Griff (1) drehbar angeordnet und eine Verriegelungseinrichtung (4) zur drehfesten Lagerung der Einrichtung (2) an dem Griff (1) vorgesehen ist.

2. Vorrichtung nach Anspruch 1 oder 2, **dadurch gekennzeichnet, dass** die Einrichtung (2) an ihrer Außenseite (12) eine Schaumstoffschicht aufweist.

3. Vorrichtung nach einem der voranstehenden Ansprüche, **dadurch gekennzeichnet, dass** die Einrichtung (2) aus Schaumstoff besteht.

4. Vorrichtung nach Anspruch 1, **dadurch gekennzeichnet, dass** der Träger (3) eine Grundfläche (31) mit einem Durchmesser von 9mm bis 11mm, bevorzugt 10mm und der Durchmesser der Einrichtung (2) 12mm bis 18mm, bevorzugt 15mm beträgt

5. Vorrichtung nach Anspruch 1, **dadurch gekennzeichnet, dass** die Verriegelungseinrichtung (4) als entlang der Grifflängserstreckung (5) verschiebliches Formschlusselement ausgebildet ist, das in Verriegelungsstellung in zumindest eine korrespondierend ausgebildete Aussparung (6) eingreift.

6. Vorrichtung nach Anspruch 1 oder 5, **dadurch gekennzeichnet, dass** Formschlusselement (4) als Abflachung, Absatz, Vorsprung oder Verzahnung im Sägezahnprofil ausgebildet ist.

7. Vorrichtung nach Anspruch 5 oder 6, **dadurch gekennzeichnet, dass** ein Federelement das Formschlusselement (4) in Entriegelungsrichtung belastet.

8. Vorrichtung nach einem der voranstehenden Ansprüche, **dadurch gekennzeichnet, dass** der Träger (3) relativ zu dem Griff (1) drehbar gelagert ist und entweder ein Formschlusselement (4) oder eine Aussparung (6) aufweist.

9. Vorrichtung nach einem der voranstehenden Ansprüche, **dadurch gekennzeichnet, dass** der Griff (1) einen eckigen Querschnitt oder einen runden Querschnitt mit einer strukturierten Oberfläche aufweist.

10. Vorrichtung nach einem der voranstehenden Ansprüche, **dadurch gekennzeichnet, dass** die Einrichtung (2) einen Schaumstoff zur Zellaufnahme mit einer Porenanzahl von 0,98 bis 1,57 ppmm, bevorzugt 1,26 bis 1,42 ppmm, besonders bevorzugt 1,34 ppmm aufweist.

11. Vorrichtung nach einem der voranstehenden Ansprüche, **dadurch gekennzeichnet, dass** die Einrichtung (2) einen Schaumstoff zur Zellaufnahme mit einer Stauchhärte von 2 bis 6 kPa, bevorzugt 3 bis 5 kPa, besonders bevorzugt 4 kPa aufweist.

12. Vorrichtung nach einem der voranstehenden Ansprüche, **dadurch gekennzeichnet, dass** die Einrichtung (2) einen Konuswinkel von 20° bis 35°, bevorzugt 25° bis 30°, besonders bevorzugt 27° aufweist.

13. Vorrichtung nach einem der voranstehenden Ansprüche, **dadurch gekennzeichnet, dass** die Stabilisierung (23) eine Länge von 85 % - 95 %, bevorzugt 87 % bis 93 %, besonders bevorzugt von 90 % der Länge der Einrichtung (2) aufweist.

14. Vorrichtung nach einem der voranstehenden Ansprüche, **dadurch gekennzeichnet, dass** der Griff (1) einen Durchmesser von 3 mm bis 8 mm, vorzugsweise 4mm bis 7 mm, besonders bevorzugt 5 mm bis 6 mm aufweist.

15. Vorrichtung nach einem der voranstehenden Ansprüche, **dadurch gekennzeichnet, dass** der Griff (1) eine Gesamtlänge (91) von 150 mm bis 250 mm, bevorzugt von 180 mm bis 220 mm, besonders bevorzugt von 200 mm aufweist.

16. Vorrichtung nach einem der voranstehenden Ansprüche, **dadurch gekennzeichnet, dass** der Griff (1) eine Sollbruchstelle (9) aufweist.

## Claims

1. Device for removing and smearing cells for a cytological examination with a handle (1) at whose front end a device (2) embodied as a cone tapering towards the front for the collection of cells is arranged, in which a stabilizing device (23) acting in the longitudinal extension of the device (2) is arranged, whereas the device (2) is embodied as a cap, that is arranged and fixed on a carrier (3) and the carrier (3) has a base surface, whose diameter is smaller than the diameter of the base surface of the device (2), whereas the stabilization (23) is embodied as a tip projecting into the device (2), which tip is surrounded on all sides by a foam material, **characterized in that** the device (2) is arranged so that it can rotate relative to the handle (1) and a locking mechanism (4) is provided for the torsionally rigid positioning of the device (2) on the handle (1).

2. Device according to claim 1 or 2, **characterized in that** the device (2) features a foam material layer on its outer side (12).

3. Device according to one of the preceding claims, **characterized in that** the device (2) is composed of foam material.

4. Device according to claim 1, **characterized in that** the carrier (3) has a base surface (31) with a diameter of 9 mm to 11 mm, preferably 10 mm and the diameter of the device (2) is 12 mm to 18 mm, preferably 15 mm.

5. Device according to claim 1, **characterized in that** the locking mechanism (4) is embodied as a positive engagement element that can be pushed along the longitudinal extension (5) of the handle, which element in the locked position engages in at least one correspondingly embodied recess (6).

6. Device according to claim 1 or 5 **characterized in that** the positive engagement element (4) is embodied as a flattening, a shoulder, a projection, or a toothing in the sawtooth profile.

7. Device according to claim 5 or 6, **characterized in that** a spring element loads the positive engagement element (4) in the unlocking direction.

8. Device according to one of the preceding claims, **characterized in that** the carrier (3) is pivoted relative to the handle (1) and features either a positive engagement element (4) or a recess (6).

9. Device according to one of the preceding claims, **characterized in that** the handle (1) features an angular cross-section or a round cross-section with a structured surface.

10. Device according to one of the preceding claims, **characterized in that** the device (2) features a foam material for the cell collection with a pore number of 0,98 to 1,57 ppmm, preferably 1,26 to 1,42 ppmm, especially preferably 1,34 ppmm.

11. Device according to one of the preceding claims, **characterized in that** the device (2) features a foam material for the cell collection with a compressive strength of 2 to 6 kPa, preferably 3 to 5 kPa, especially preferably 4 kPa.

12. Device according to one of the preceding claims, **characterized in that** the device (2) features a cone angle of 20° to 35°, preferably 25° to 30°, especially preferably 27°.

13. Device according to one of the preceding claims, **characterized in that** the stabilization (23) features a length of 85 % - 95 %, preferably 87 % to 93 %, especially preferably 90 %, of the length of the device (2).

14. Device according to one of the preceding claims, **characterized in that** the handle (1) features a diameter of 3 mm to 8 mm, preferably 4 mm to 7 mm, especially preferably 5 mm to 6 mm.

15. Device according to one of the preceding claims, **characterized in that** the handle (1) features a total length (91) of 150 mm to 250 mm, preferably 180 mm to 220 mm, especially preferably 200 mm.

16. Device according to one of the preceding claims, **characterized in that** the handle (1) features a predetermined breaking point (9).

## Revendications

1. Dispositif de prélèvement et d'étalement de cellules pour un examen cytologique comprenant un manche (1) à l'extrémité avant duquel est disposé un organe (2) en forme de cône se rétrécissant vers l'avant pour recevoir les cellules, dans lequel est agencé dans la direction de la longueur de l'organe (2) un organe actif de stabilisation (23), l'organe (2) étant à cet effet réalisé sous forme de capuchon étant à cet effet réalisé sous forme de capuchon qui est disposé et fixé sur un support (3) et le support (3) comportant une surface de base dont le diamètre est inférieur au diamètre de la surface de base de l'organe (2), l'organe de stabilisation (23) étant réalisé sous forme de pointe engagée dans l'organe (2) qui est entourée de tous les côtés par un produit mousseux, **caractérisé en ce que** l'organe (2) est agencé de façon rotatif par rapport au manche (1) et un organe de verrouillage (4) est prévu pour positionner de façon fixe l'organe (2) par rapport au manche (1).

2. Dispositif selon la revendication 1 ou 2, **caractérisé en ce que** l'organe (2) présente sur sa surface extérieure (12) une couche de mousse.

3. Dispositif selon l'une des revendications précédentes, **caractérisé en ce que** l'organe (2) est constitué de mousse.

4. Dispositif selon la revendication 1, **caractérisé en ce que** le support (3) comporte une surface de base (31) ayant un diamètre de 9mm à 11mm, de préférence 10mm et le diamètre de l'organe (2) est compris entre 12mm et 18mm, de préférence 15mm.

5. Dispositif selon la revendication 1, **caractérisé en ce que** l'organe de verrouillage (4) est réalisé sous forme d'élément de verrouillage à coopération de formes pouvant coulisser le long de l'extension longitudinale (5) du manche, qui, dans la position de verrouillage vient en prise dans au moins un évidement correspondant (6).

6. Dispositif selon la revendication 1 ou 5, **caractérisé en ce que** l'élément de verrouillage à coopération de formes (4) est réalisé sous forme de méplat, renfoncement, saillie ou dentelure à profil en dent de scie.

7. Dispositif selon la revendication 5 ou 6, **caractérisé en ce qu'**un élément ressort sollicite l'élément de verrouillage à coopération de formes (4) dans la direction de déverrouillage.

8. Dispositif selon l'une des revendications précédentes, **caractérisé en ce que** le support (3) est monté de façon rotative par rapport au manche (1) et présente soit un élément de verrouillage à coopération de formes (4) ou un évidemment.

9. Dispositif selon l'une des revendications précédentes, **caractérisé en ce que** le manche (1) présente une section soit anguleuse soit une section circulaire ayant une surface structurée.

10. Dispositif selon l'une des revendications précédentes, **caractérisé en ce que** l'organe (2) comporte une mousse pour la réception des cellules ayant un nombre de pores compris entre 0,98 et 1,57 ppmm, de préférence 1,26 à 1,42ppmm, de façon particulièrement préférée 1,34ppmm.

11. Dispositif selon l'une des revendications précédentes, **caractérisé en ce que** l'organe (2) est une mousse pour la réception des cellules ayant une dureté au refoulement de 2 à 6kPa, de préférence de 3 à 5kPa, de façon particulièrement préférée de 4kPa.

12. Dispositif selon l'une des revendications précédentes, **caractérisé en ce que** l'organe (2) présente un angle de cône de 20° à 35°, de préférence de 25° à 30° et de façon particulièrement préférée de 27°.

13. Dispositif selon l'une des revendications précédentes, **caractérisé en ce que** l'organe de stabilisation (23) présente une longueur égale à 85% - 95%, de préférence 87% à 93%, de façon particulièrement préférée de 90% de la longueur de l'organe (2).

14. Dispositif selon l'une des revendications précédentes, **caractérisé en ce que** le manche (1) présente un diamètre de 3mm à 8mm, de préférence 4mm à 7mm et de façon particulièrement préférée de 5mm à 6mm.

15. Dispositif selon l'une des revendications précédentes, **caractérisé en ce que** le manche (1) présente une longueur totale (91) de 150mm à 250mm, de préférence de 180mm à 220mm, de façon particulièrement préférée de 200mm.

16. Dispositif selon l'une des revendications précédentes, **caractérisé en ce que** le manche (1) présente une zone de rupture (9).
